# NEW EUROPEAN PATENT SPECIFICATION

(11) **EP 0 585 939 B2**
(45) Date of publication and mention of the opposition decision: **02.04.2008**
(45) Mention of the grant of the patent: 30.01.2002
(21) Application number: 93114141.0
(22) Date of filing: 03.09.1993
(51) Int. Cl.: C12N 15/13, C12P 21/08, C07K 16/28, C07K 16/42, A61K 39/395, C12N 15/12, C12N 15/62

(54) **TNF ligands**
TNF-Liganden
Ligands du TNF

(30) Priority: 03.09.1992 IL 103051; 08.07.1993 IL 106271
(43) Date of publication of application: 09.03.1994
(73) Proprietor: YEDA RESEARCH AND DEVELOPMENT CO., LTD., 76100 Rehovot (IL)
(72) Inventor: Wallach, David, Rehovot (IL); Bigda, Jacek, 80-809 Gdansk (PL); Beletsky, Igor, RAS, Puschino 142292 (RU); Mett, Igor, Rehovot (IL)
(74) Representative: Vossius & Partner

(56) References cited:
- EP-A- 0 398 327
- EP-A- 0 444 560
- JOURNAL OF BIOLOGICAL CHEMISTRY., vol.267, no.9, 25 March 1992, BALTIMORE US pages 5747 - 5750 MARSTERS, S.A. ET AL.; 'Identification of cysteine-rich domains of the type 1 tumor necrosis factor receptor involved in ligand binding'

## Description

### FIELD OF THE INVENTION

The present invention relates to the use of ligands to Tumor Necrosis Factor receptors (TNF-Rs) which inhibit the effect of TNF but not its binding to the TNF-Rs, as well as to ligands interacting with other receptors of the TNF/NGF receptor family. These ligands are antibodies, peptides derived therefrom or a salt or mutein of said antibodies. According to the invention the ligands are used for the preparation of a pharmaceutical composition for the treatment of septic shock, cachexia, graft-versus-host disease and autoimmune diseases, in particular rheumathoid arthritis.

### BACKGROUND OF THE INVENTION

Tumor necrosis factor (TNF) is a pleiotropic cytokine, produced by a number of cell types, mainly by activated macrophages. It is one of the principal mediators of the immune and inflammatory response. Interest in its function has greatly increased, recently, in view of evidence of the involvement of TNF in the pathogenesis of a wide range of disease states, including endotoxin shock, cerebral malaria and graft-versus-host reaction. Since many of the effects of TNF are deleterious to the organism, it is of great interest to find ways of blocking its action on host cells. An evident target for such intervention are the molecules to which TNF has to bind in order to exert its effects, namely the TNF-Rs. These molecules exist not only in cell-bound, but also in soluble forms, consisting of the cleaved extra-cellular domains of the intact receptors (see Nophar et al., EMBO Journal, 9(10):3269-78, 1990). The soluble receptors maintain the ability to bind TNF, and thus have the ability to block its function by competition with surface receptors.

Another method of TNF inhibition based on the principle of competing with cell-bound molecules, is the use of antibodies recognizing TNF receptors and blocking the ligand binding.

The cell surface TNF-Rs are expressed in almost all cells of the body. The various effects of TNF, the cytotoxic, growth-promoting and others, are all signalled by the TNF receptors upon the binding of TNF to them. Two forms of these receptors, which differ in molecular size: 55 and 75 kilodaltons, have been described, and will be called herein p55 and p75 TNF-R, respectively. It should be noted, however, that there exist publications which refer to these receptors also as p60 and p80.

The TNF-Rs belong to a family of receptors which are involved in other critical biological processes. Examples of these receptors are the low affinity NGF receptor, which plays an important role in the regulation of growth and differentiation of nerve cells. Several other receptors are involved in the regulation of lymphocyte growth, such as CDw40 and some others. Another member of the family is the FAS receptor also called APO, a receptor which is involved in signalling for apoptosis and which, based on a study with mice deficient in its function, seems to play an important role in the etiology of a lupus-like disease. Herein, this family of receptors is called "TNF/NGF receptor family".

One of the most striking features of TNF compared to other cytokines, thought to contribute to the pathogenesis of several diseases, is its ability to elicit cell death. The cell-killing activity of TNF is thought to be induced by the p55 receptor. However, this p55 receptor activity can be assisted by the p75 receptor, through a yet unknown mechanism.

European patent publication no.s 0,398,327 and 0,412,486 disclose antibodies to the soluble TNF-Rs. These antibodies were found to recognize the soluble TNF-Rs and to inhibit the binding of TNF to the TNF-Rs on the cell surface. Monovalent F(ab) fragments blocked the effect of TNF, while intact antibodies were observed to mimic the cytotoxic effect of TNF.

Marsters et al. (J. Biol. Chem. 267 (1992), 5747-5750) describe the identification of cysteine-rich domains of the type 1 tumor necrosis factor receptor involved in ligand binding and also describe a monoclonal antibody binding to the TNF-receptor 1. However, this antibody is not capable of inducing a conformational change of the receptor in contrast to the ligands of the present application. Moreover, it is not conclusively shown that the monoclonal antibodies actually interact with the region of the TNF-receptor 1 corresponding to CDR4. EP-A1 0 444 560 discloses particular antibodies against a TNF-receptor. However, EP-A1 0 444 560 does not disclose antibodies having the features of the antibodies of the present application, for example it does not disclose antibodies binding to the region of the TNF-receptor which corresponds to the region which the ligands of the present application bind to. It is even not clear from EP-A1 0 444 560 whether the antibodies are directed against the extracellular epitope of the receptor.

### SUMMARY OF THE INVENTION

The present invention provides the use of a ligand as characterized in the claims to a member of the TNF/NGF receptor family, which binds to the region of the C-terminal cysteine loop of such a receptor and which is inhibitory to the signaling for the cytocidal effect of said receptor, wherein the cysteine loop includes the amino acid sequence cys-163 to thr-179 in the p75 TNF-R or a corresponding C-terminal cysteine loop in another member of the TNF/NGF family.

Preferably, the receptor is the TNF-R, in particular the p75 TNF-R.

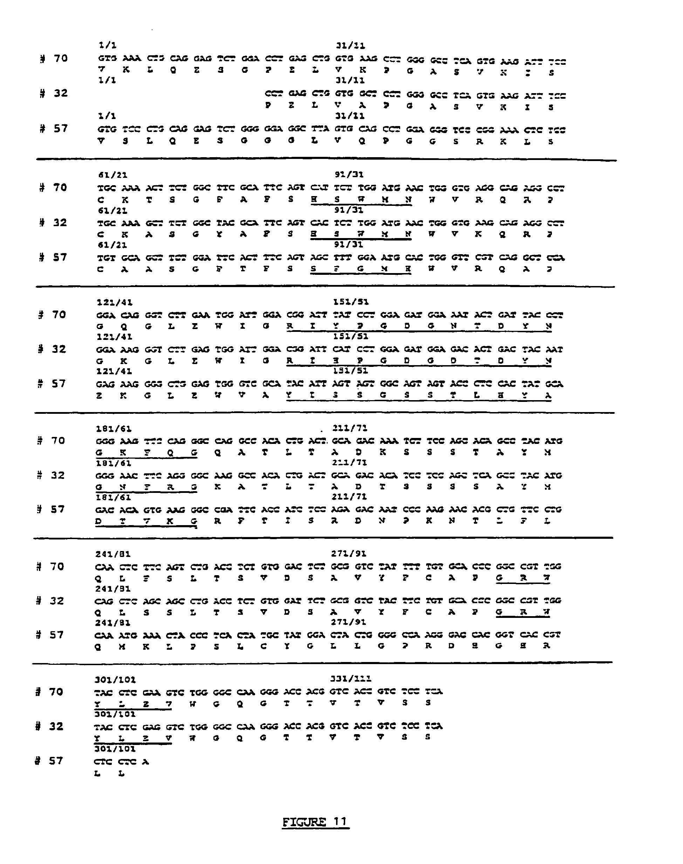

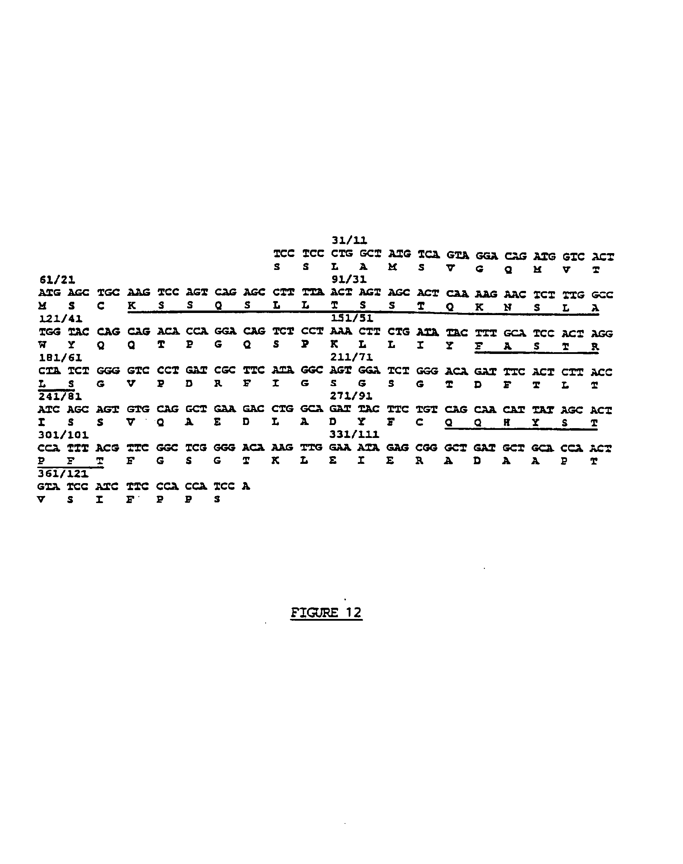

One such ligand includes the amino acid sequence for the CDR region of the heavy chain of monoclonal antibody no. 32, shown in Fig. 11, and/or the amino acid sequence for the CDR region of the light chain of this antibody shown in Fig. 12.

Another such ligand includes the amino acid sequence for the CDR region of the heavy chain of monoclonal antibody no. 70 shown in Fig. 11.

Yet another such ligand includes the amino acid sequence for the CDR region of the heavy chain of monoclonal antibody no. 57, shown in Fig. 11.

The above antibodies are called herein, for simplicity's sake, "group 32" antibodies.

In another aspect of the invention, the ligands comprise the scFv of a group 32 antibody.

The ligands are those which are characterized in the claims.

The ligand may be comprised in a fusion protein with another protein, optionally linked by a peptide linker. Such a fusion protein can increase the retention time of the ligand in the body, and thus may even allow the ligand-protein complex to be employed as a latent agent or as a vaccine.

The peptides of the invention can be synthesized by various methods which are known in principle, namely by chemical coupling methods (cf. Wunsch, E: "Methoden der organischen Chemie", Volume 15, Band 1 + 2, Synthese von Peptiden, thime Verlag, Stuttgart (1974), and Barrany, G.; Marrifield, R.B.: "The Peptides", eds. E. Gross, J. Meienhofer, Volume 2, Chapter 1, pp. 1-284, Academic Press (1980)), or by enzymatic coupling methods (cf. Widmer, F. Johansen, J.T., Carlsberg Res. Commun., Vol.44, pp. 37-46 (1979), and Kullmann, W.: "Enzymatic Peptide Synthesis" CRC Press Inc. Boca Raton, Fl. (1987), and Widmer, F., Johansen, J.T. in "Synthetic Peptides in Biology and Medicines:, eds. Alitalo, K., Partanen, P., Vatieri, A., pp.79-86, Elsevier, Amsterdam (1985)), or by a combination of chemical and enzymatic methods if this is advantageous for the process design and economy.

A cysteine residue may be added at both the amino and carboxy terminals of the peptide, which will allow the cyclisation of the peptide by the formation of a di-sulphide bond.

Any modifications to the peptides of the present invention which do not result in a decrease in biological activity are within the scope of the present invention.

The individual specificity of antibodies resides in the structures of the peptide loops making up the Complementary Determining Regions (CDRs) of the variable domains of the antibodies.

All of the molecules (peptides, etc.) may be produced either by conventional chemical methods, as described herein, or by recombinant DNA methods.

The invention also provides DNA molecules encoding the ligands according to the invention, vectors containing them and host cells comprising the vectors and capable of expressing the ligands according to the invention.

The host cell may be either prokaryotic or eukaryotic.

The invention further provides DNA molecules hybridizing to the above DNA molecules and encoding ligands having the same activity.

The invention also provides pharmaceutical compositions comprising the above ligands which are useful for treating diseases induced or caused by the effects of TNF, either endogenously produced or exogenously administered.

### BRIEF DESCRIPTION OF THE FIGURES

Figure 1 shows a diagrammatic illustration of the bacterial constructs used for determining the sequence to which antibodies of the 32 group bind.

Figure 2 shows an example of the Western blotting analysis technique by which the binding of the antibodies to the constructs shown in Figure 1 have been determined.

Figures 3 & 4 show the competition of synthetic peptides whose sequences contain the region of the epitope recognized by the monoclonal antibodies of the 32 group, or parts of it, with the binding of an antibody of this group to a construct comprising part of TBP-II in which this epitope is present.

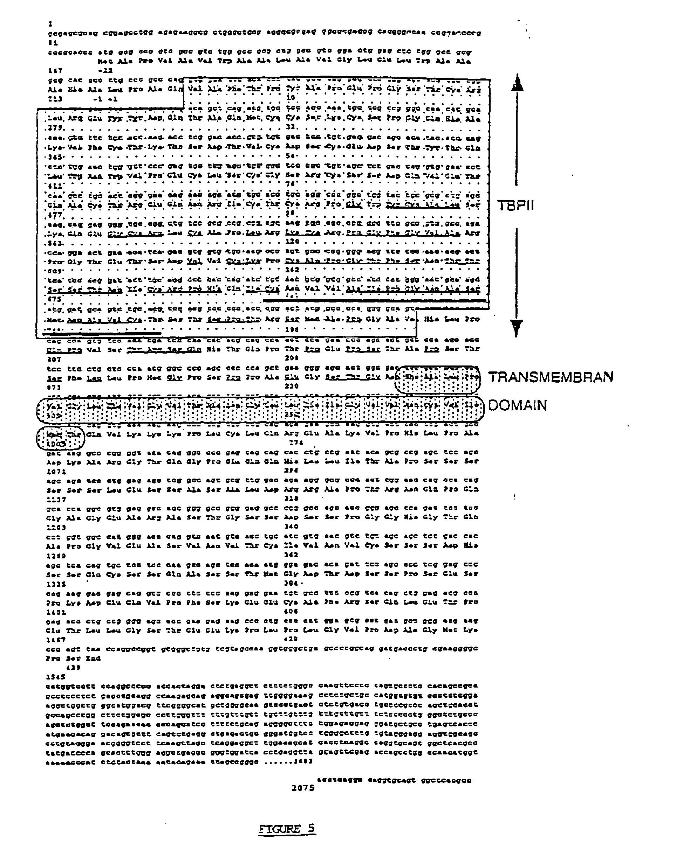

Figure 5 shows the nucleotide and deduced amino acid sequences of the p75 receptor. TBP-II and transmembranal domains are boxed and shaded. The region recognized by the group 32 antibodies is underlined.

Figure 6 shows the pattern of protection of HeLa p75.3 cells (as hereinafter defined) from TNF cytotoxicity by different monoclonal antibodies against p75 TNF-R, and fragments thereof.

Figure 7 shows the effects of a monoclonal antibody against TBP-I and several against TBP-II on the extent of killing of U937 cells by TNF.

Figures 8a and 8b show the effects of monoclonal antibody 70 and Fab fragments thereof on the binding of TNF to HeLa p75.3 cells and U937 cells, respectively.

Figure 9 shows a comparison of the effects of the antibody 32 with other antibodies against the p75 TNF-R on TNF binding to HeLa p75.3 cells.

Figure 10 shows dissociation of TNF from HeLa p75.3 cells in the presence and absence of antibody no. 70 and its monovalent Fab fragment.

Figure 11 shows the nucleotide and deduced amino acid sequences for the CDR region of the heavy chains of three monoclonal antibodies of the 32 group.

Figure 12 shows the nucleotide and deduced amino acid sequences for the CDR region of the light chains of monoclonal antibody No. 32.

Figure 13 shows the sequence homology between several members of the TNF/NGF receptor family.

### DETAILED DESCRIPTION OF THE INVENTION

TNF, as stated above, is a cytokine which initiates its effect on cell function by binding to two specific cell surface receptors: the p55 and p75 receptors. Binding of antibodies to the extracellular domain of these receptors can interfere with its effect. However, as shown in a number of studies, antibodies binding to the extracellular domain of the receptors can also trigger the effects of TNF by inducing aggregation of the p55 receptors, as well as by inducing aggregation of the p75 receptors. (Engelmann, et al. J. Biol. Chem., Vo. 265, No. 24, pp. 14497-14504, 1990; and unpublished data).

We have found that certain antibodies binding to one particular region in the p75 receptor are not mimetic but rather inhibitory to the signalling for the cytocidal effect by this receptor. This, in spite of the fact that when binding to this region, these antibodies do not block TNF binding, but rather increase it to some extent.

The present invention reveals that this region recognized by these antibodies which we call the 32 group, is the region extending between the two C-terminal cysteines in the extracellular domain of the p75 receptor, plus an additional amino acid, thr179. This region for simplicity's sake, is called "cysteine loop" throughout this specification.

The present invention also provides the nucleotide sequences and deduced amino acid sequences in the CDR of the heavy chain of the three antibodies belonging to this group, named 32 and 57. In addition, the invention also provides the nucleotide sequence and the deduced amino acid sequence of the light chain of antibody 32. Based on these sequences, peptides which will inhibit the function of the p75 receptors can be defined.

In view of these findings, as well as the close similarity of the receptors in this particular family, this invention relates also to agents which bind to the C-terminal cysteine loop in the extracellular domain of the various other members of the TNF/NGF receptor family and modulate the function of the other receptors, similarly to the modulation of the function of TNF. In this receptor family, the localization of cysteine in the extra-cellular domain and the spacing is highly conserved. Certain members of this family, e.g. CDw40, exhibit particularly high similarity to the p75 receptor. Particularly in such receptors, agents binding to these regions are expected to have effects similar to the effect of the 32 antibodies on the p75 receptor.

The active molecules obtained by the above procedures, inasfar as they are biological substances, can also be prepared by biotechnological approaches. In this way, massive production of these molecules will be made possible. Peptides may either be produced by known peptide synthesis methods or using expression vectors containing DNA sequences encoding them. Other molecules, if produced in an enzymatic way, can be made by producing the enzymes involved in the appropriate cultured cells.

Pharmaceutical compositions containing the ligands of the present invention may be employed for antagonizing the effects of TNF in mammals.

Such compositions comprise the ligands according to the invention as their active ingredient. The pharmaceutical compositions are indicated for conditions such as septic shock, cachexia, graft-versus-host reactions, autoimmune diseases such as rheumatoid arthritis, and the like. They are also indicated for counteracting e.g. an overdose of exogenously administered TNF.

The pharmaceutical compositions according to the invention are administered depending on the condition to be treated, via the accepted ways of administration. For example, in the case of septic shock, intravenous administration will be preferred. The pharmaceutical compositions may also be administered continuously, i.e. by way of infusion, or orally. The formulation and dose will depend on the condition to be treated, the route of administration and the condition and the body weight of the patient to be treated. The exact dose will be determined by the attending physician.

The pharmaceutical compositions according to the invention are prepared in the usual manner, for example by mixing the active ingredient with pharmaceutically and physiologically acceptable carriers and/or stabilizers and/or excipients, as the case may be, and are prepared in dosage form, e.g. by lyophilization in dosage vials.

As used herein the term "muteins" refers to analogs of the ligands in which one or more of the amino acid residues of the ligand found to bind are replaced by different amino acid residues or are deleted, or one or more amino acid residues are added to the original sequence, without changing considerably the activity of the resulting product. These muteins are prepared by known synthesis and/or by site-directed mutagenesis techniques, or any other known technique suitable therefor.

The term "fused protein" refers to a polypeptide comprising the ligands or a mutein thereof fused with another protein which has an extended residence time in body fluids. The ligands may thus be fused to another protein, polypeptide or the like, e.g. an immunoglobulin or a fragment thereof.

The term "salts" herein refers to both salts of carboxyl groups and to acid addition salts of amino groups of the ligands, muteins and fused proteins thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid.

Derivatives include polyethylene glycol side-chains which may mask antigenic sites and extend the residence of the ligands in body fluids.

The invention is illustrated by the following non-limiting examples:

### EXAMPLE 1: Determination of the region of the p75 receptor which is recognized by the group 32 antibodies

In example 5 of the main application, a number of constructs were prepared by expression in E. coli, and it was concluded that the epitope recognized by antibody no. 32 maps between amino acids 125-182.

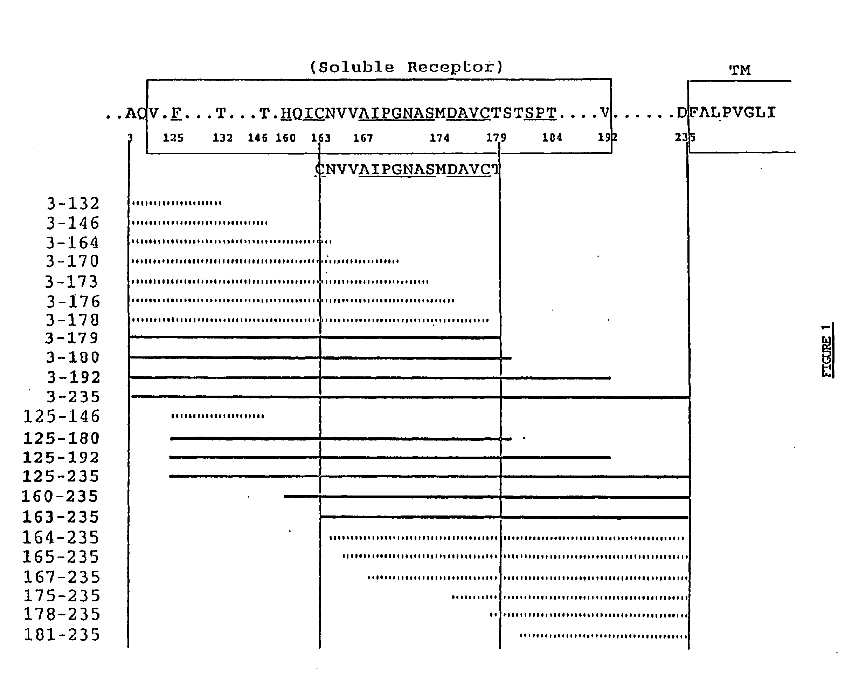

We have now prepared further constructs and the complete list of constructs examined, as well as their relationship to the structure of the soluble p75R are shown in Fig. 1. Constructs recognized by the antibodies of the 32 group are listed in bold numbers and illustrated as solid lines. Those not reacting with these antibodies are listed in thin numbers and illustrated by broken lines. All constructs are identified by their N- and C-terminal amino acid residues.

Figure 1, above the diagrammatic illustration of the constructs, shows the amino acid sequence of part of the p75 TNF-R, the regions corresponding to the soluble form of the receptor and the transmembranal region being boxed. Amino acid residues conserved between man and mouse are underlined.

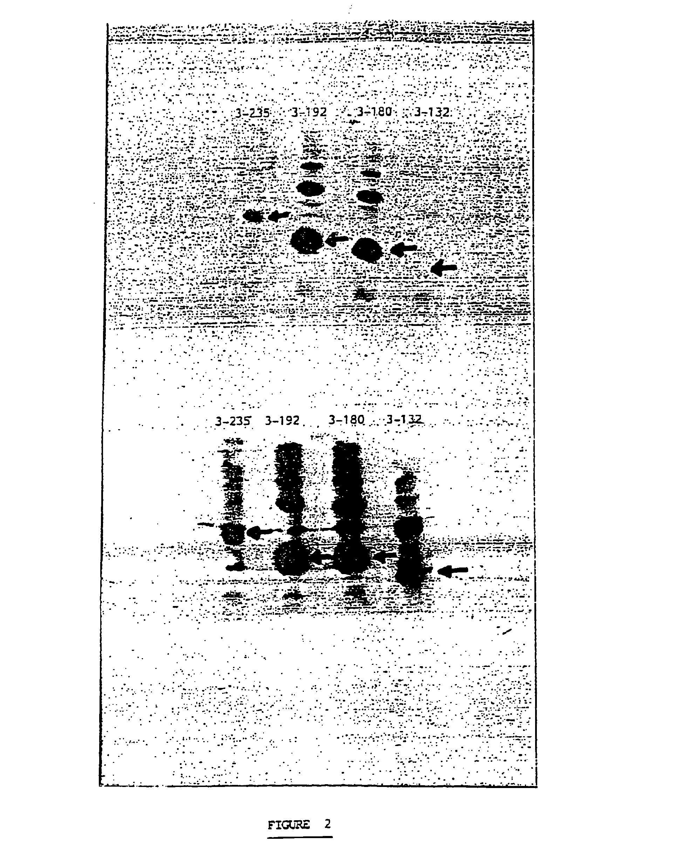

The Western blotting analysis shown in Figure 2, of the binding of the group 32 antibodies to some of the constructs shown in Figure 1 was carried out as in Example 5 of the main application.

### EXAMPLE 2: Competition for binding to the extracellular domain of the p75 TNF-R between group 32 antibodies and synthetic peptides

A number of synthetic peptides whose sequences correspond to various parts of the region on the TNF-R suspected to be the group 32 epitope were synthetized (residues 160-179, 162-179, 163-179, 165-179 and 167-179). The peptides were examined in an ELISA test for their ability to compete for the binding to the antibodies of the 32 group.

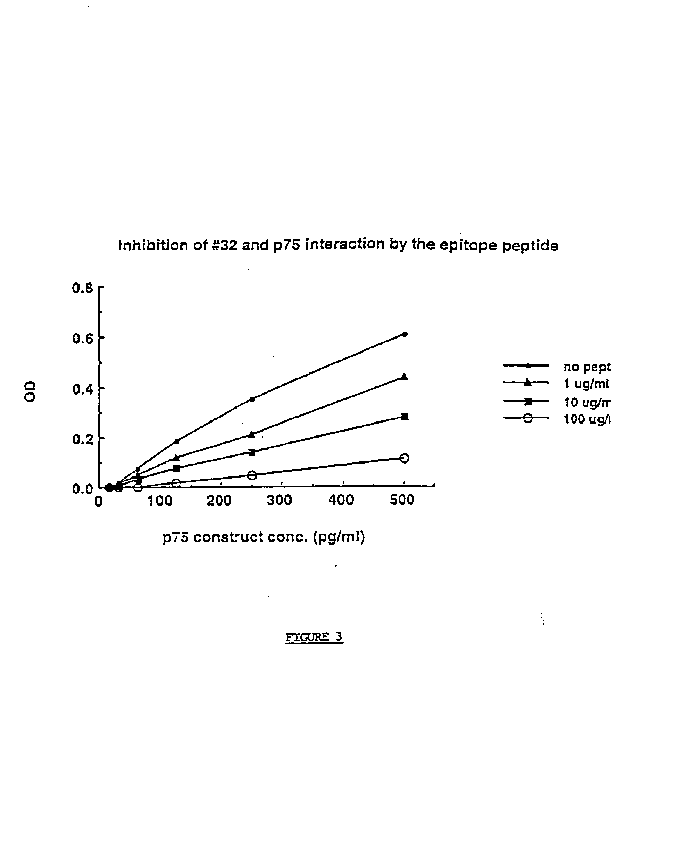

A bacterially produced construct corresponding to amino acids 3 to 180 of the p75 TNF-R (p75 construct in Fig. 3) was applied, at the indicated concentrations, to PVC plates precoated with antibody 32 followed by application of rabbit antiserum to TBP-II (p75 soluble TNF-R). The amount of rabbit antiserum bound to the plate was determined by applying goat antiserum against rabbit immunoglobulin, coupled to horse-radish peroxidase and enzymatic assessment of the amount of goat immunoglobulin bound to the plate. Figure 3 shows the data of an experiment in which a synthetic peptide corresponding to amino acid residues 163 to 179 was found to compete for the binding.

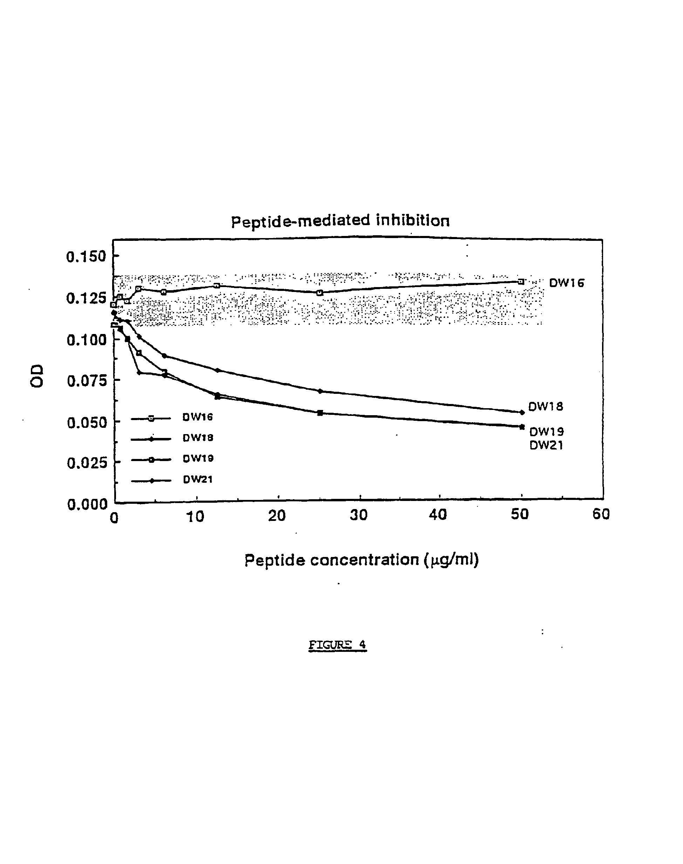

Figure 4 shows the data of an experiment in which a fusion protein of maltose binding protein (MBP) with the sequence of amino acids extending from 125 to 192 of the p75 receptor was used to coat PVC plates at a concentration of 10µg/ml, then the No. 32 McAb was applied at a concentration of 2µg/ml together with the indicated concentrations of different peptides:
DW16 - amino acids 165-179
DW18 - amino acids 163-179
DW19 - amino acids 162-179
DW21 - amino acids 160-179

Thereafter, the reaction was developed by adding goat anti-mouse coupled to horseradish peroxidase. As shown in Fig. 4, marked inhibition of fusion protein recognition by monoclonal antibody No. 32 was observed only with the three peptides covering the whole epitope.

### EXAMPLE 3: Mutational study of the 32 epitope

Replacing cysteine 178 with alanine in a recombinant peptide whose sequence corresponds to amino acids 3 to 181, made this protein unrecognizable by the 32 group antibodies. This finding suggests that in order to be recognized by these antibodies, the two cysteines in the group 32 epitope region must be free to interact with each other; i.e. that the structure recognized by the antibodies is a loop. In support of this notion, we found that reduction of the peptide with dithiothreithol prior to SDS PAGE and Western blotting analysis somewhat decreased the effectivity of its recognition by the 32 group antibodies, and reduction by dithiothreithol followed by alkylation with iodoacetimide made it completely unrecognizable by the antibodies.

### EXAMPLE 4: Effects of various antibodies and fragments thereof on TNF toxicity

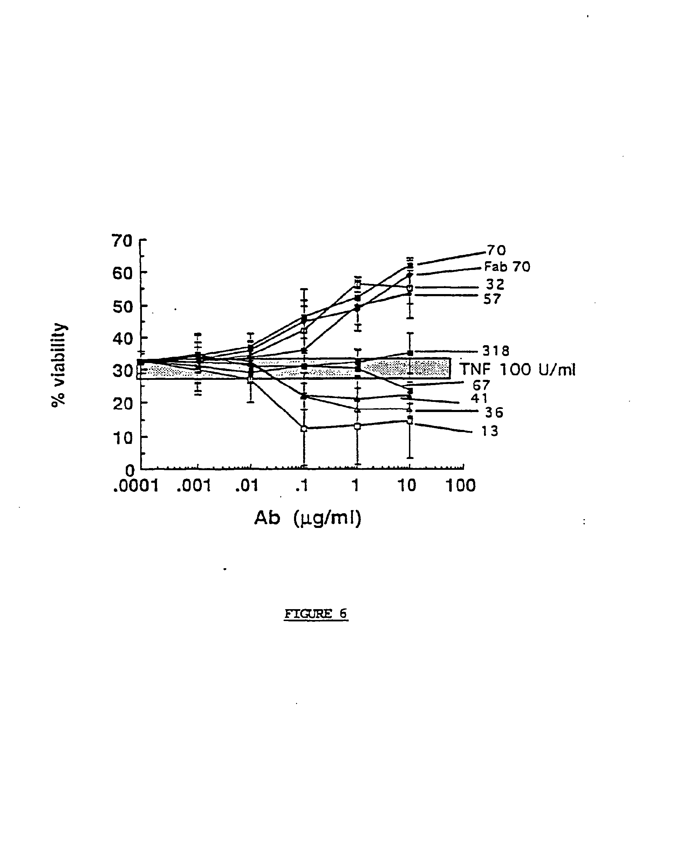

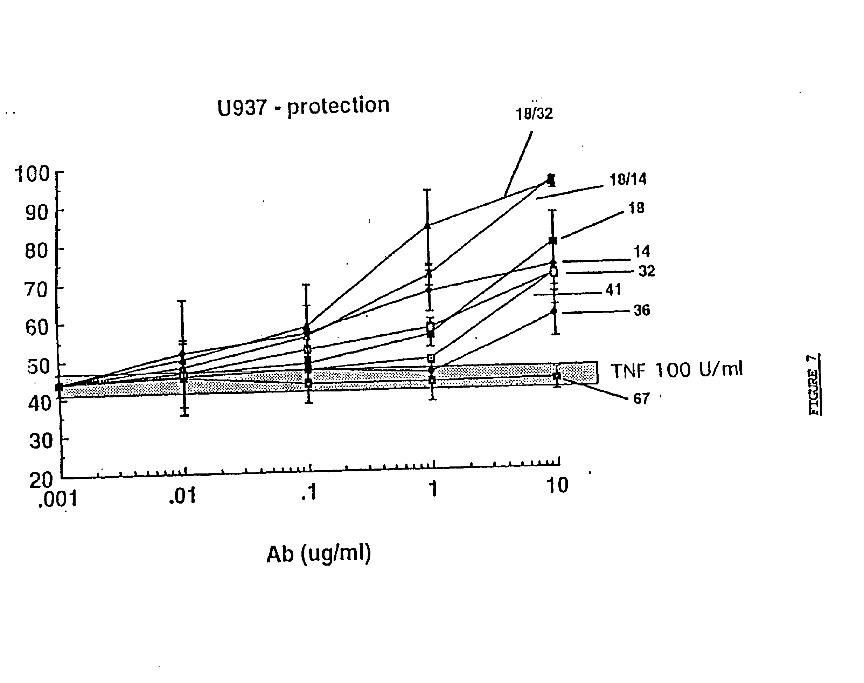

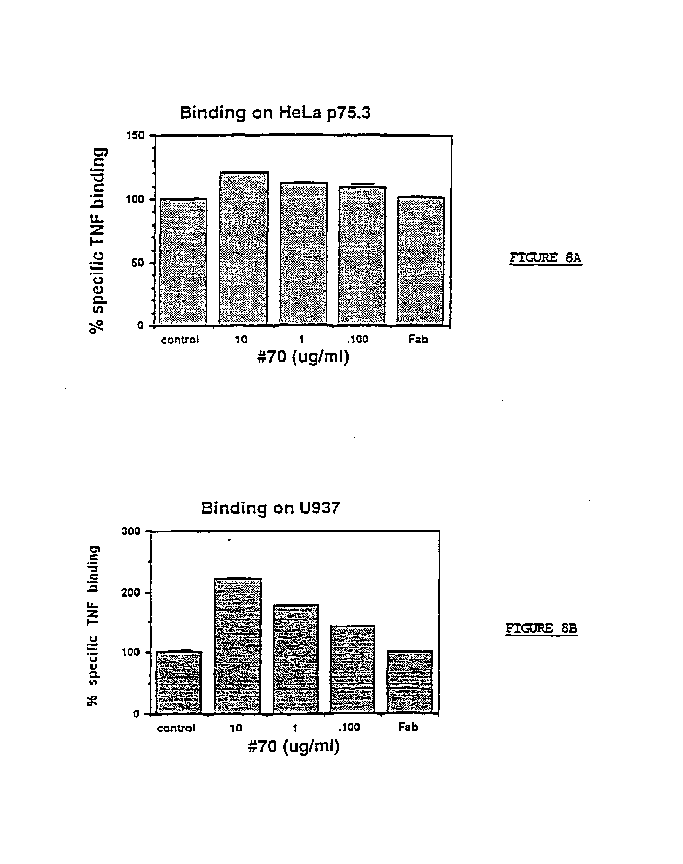

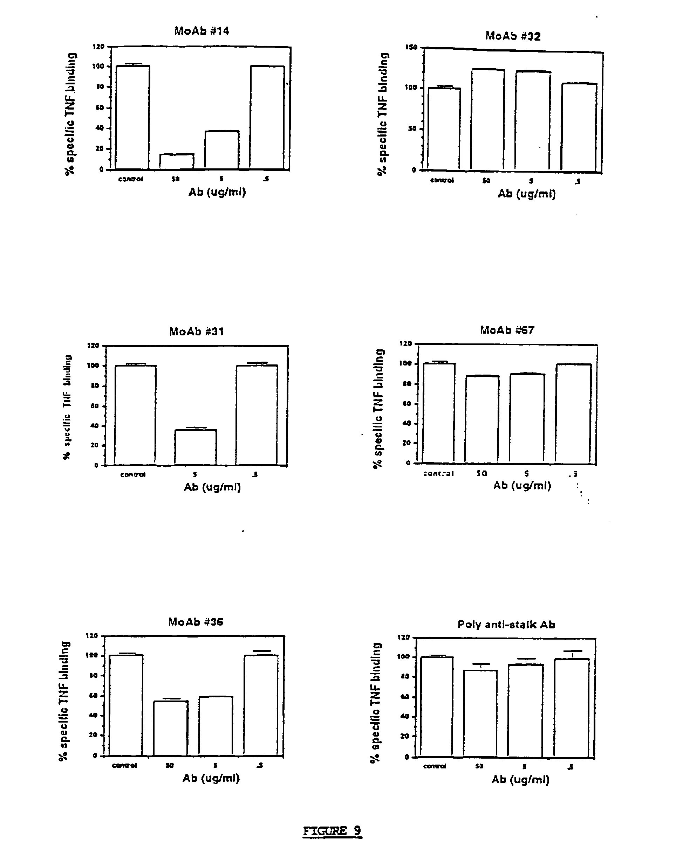

(a) In order to compare the function of the 32 group antibodies, not only to antibodies which bind to the receptor upstream to the 32 epitope region (as most of the anti-TBP-II antibodies are expected to), but also to antibodies that bind to the receptor downstream to that epitope region, we immunized mice with a chimeric construct corresponding to the region extending downstream to the 32 epitope (amino acids 181 to 235; the "stalk" region), linked to MBP. The rabbits developed antibodies which bound to the chimera with which they were immunized as well as to the intact p55 TNF receptor. These antibodies were affinity purified by binding to the chimeric protein, linked to an Affi-gel 10 column, and tested for effect on TNF function and binding. (The affinity purified antibody preparation was termed "318").
(b) All monoclonal anti-TBP-II antibodies as well as the affinity purified antistalk antibodies were tested for effect on TNF toxicity in clones of the epitheloid HeLa cells which were made to over-express the p75 receptors (by their transfection with the p75 receptor's cDNA. We called the particular over-expressing clone used in the experiments presented here, HeLa p75.3). The only antibodies found to inhibit TNF function were the antibodies of the group 32 epitope; that, in spite of the fact that they do not inhibit, but somewhat: increase TNF binding to the receptor (Figs. 8 and 9). Two of the other anti-TBP-II antibodies (No. 67 of Figs. 6 and 9 and number 81) had very little effect on TNF binding to the receptor or on TNF toxicity. All other monoclonal anti-TBP-II antibodies somewhat potentiated the cytocidal effect of TNF even though competing with TNF binding (e.g. antibody 36 of Figs 6 and 9). The "anti-stalk" antibodies had very little effect on TNF binding or function (Figs. 6 and 9). Applying the anti-stalk antibodies on the cells together with antibodies of the 32 group did not interfere with the inhibitory effect of the latter on TNF function.
(c) The same panel of antibodies was tested for effect on the killing of the myelocytic U937 cells by TNF. As opposed to the mimetic effect of anti-TNF receptor antibodies in the HeLa cells, neither anti-p55 nor anti-p75 receptor antibodies were found to be mimetic to the cytocidal effect of TNF on the U937 cells under the conditions of the experiment carried out. Having no ability to mimic the effect of TNF, all monoclonal antibodies which compete for TNF binding either to the p75 receptor, (e.g. antibodies 14, 31 and 36 of Figure 9) or to the p55 receptor (e.g. antibody number 18 of Figure 7) are inhibitory to the TNF effects. Antibodies which had no effect on TNF binding to the receptors (e.g. number 67 of Figure 9) had no effect on TNF function (Figure 6). The 32 group antibodies were unique in having an ability to inhibit TNF function in this cell without having any inhibitory effect on TNF binding. The antibodies actually enhanced the binding of TNF to these cells, much more so than in the HeLa p75.3 cells (Figure 8). The inhibitory effect of the 32 group antibodies was additive to that of antibodies which block TNF binding to the p55 receptor (e.g. the combination 18/32 in Figure 7).

### EXAMPLE 5: Effect of group 32 antibodies and Fab monovalent fragments thereof on the dissociation of TNF from the TNF-Rs

In order to explore the mechanism by which the 32 group antibodies cause an increase in TNF binding, we compared the rate of TNF dissociation from HeLa p75.3 cells in the presence and absence of these antibodies.

Radiolabelled TNF was added to confluent HeLa p75.3 cells and the cells were incubated for 2 hr on ice. Unbound ligand was washed away and 1 ml of binding buffer containg 500 ng/ml of cold TNF was applied into quadruplicate wells for the indicated periods of time on ice. Thereafter, the wells were washed once again with cold PBS, and amount of residual ligand was determined by measuring radioactivity of cells detached from plates by incubation with PBS/EDTA solution. The antibodies were applied throughout the assay at a concentration of 10µg/ml.

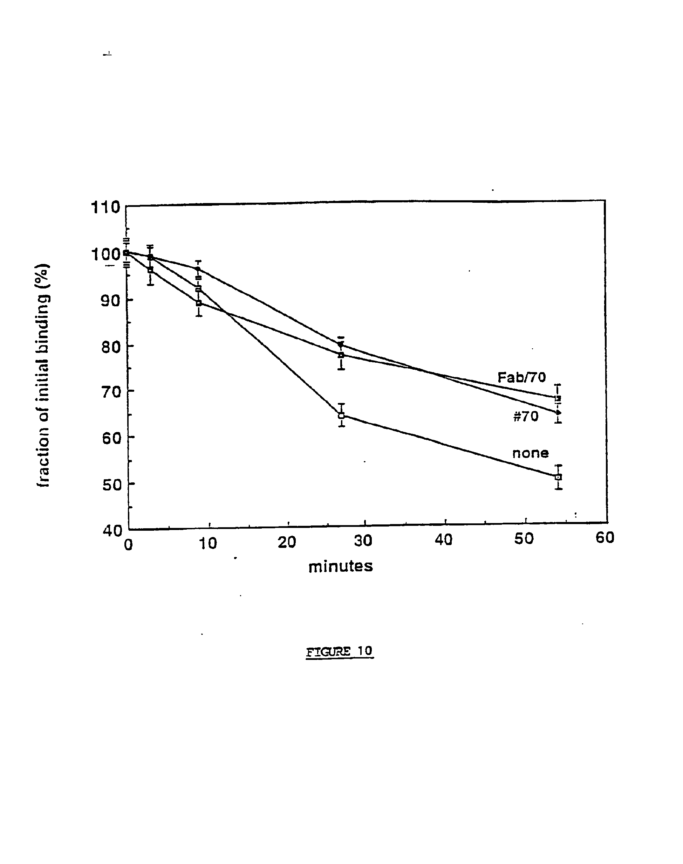

As illustrated in Fig. 10, both these antibodies as well as their F(ab) monovalent fragments caused a decrease in the rate of TNF dissociation from the receptors. Besides providing a possible explanation for the way in which these antibodies affect TNF binding to its receptors, this finding indicated an additional application for this effect. Soluble forms of the p75 TNF-Rs or of the p55 receptor or of any other member of the TNF/BGF receptor family in which a conformational change as that imposed by the 32 group antibody will occur, will serve as better inhibitors of the respective agonist.

### EXAMPLE 6: Determination of nucleotide sequences and deduced amino acid sequences in the CDR of the heavy chains of monoclonal antibodies 32, 57 and 70 (Group 32 antibodies) and in the CDR of the light (Kappa) chain of antibody 32

In order to determine the nucleotide sequences of the CDR of the heavy chains of antibodies 32, 57 and 70, total RNA was isolated by the Promega protocol from the respective hybridoma cells, with the use of guanidinium thioisocyanate. First strand cDNA synthesis on this RNA was performed with the use of AMV reverse transcriptase and either oligo(dT)15-18 or an oligonucleotide complementary to the constant region of the heavy chain of murine IgG as a primer. The cDNA was used as a template for PCR, applying a partially degenerate 5'-Primer. 40 cycles of PCR were carried out. PCR products with the size of about 350 bp were purified electrophoretically and cloned into the Bluescript vector. Clones having inserts of the right size were sequenced. Double-stranded cDNA of the CDR region of the light chain of antibody no. 32 was synthesized in a similar manner.

The nucleotide sequences obtained by the dideoxy chain termination method, and the amino acid sequences deduced therefrom are shown in Figures 11 and 12. The CDR1, 2 and 3 regions are underlined.

### EXAMPLE 7: Preparation of scFv of the 32 group antibodies

The cloned variable regions of the heavy and light chains of the monoclonal antibodies of the 32 group are linked with a linker of 15 amino acid length and introduced into a commercial expression vector. The vector contains a promoter, e.g. lac, a leader sequence e.g. pel-B, as well as a sequence encoding a small peptide ("tag" peptide) against which a monoclonal antibody is commercially available. The plasmid is now introduced into E. coli and the bacteria are grown to O.D. 0.5-1.0. Expression of scFv is induced by addition of IPTG and growth is continued for another 6-24 hrs. The soluble scFv-tag complex is then isolated from the culture medium by immunoaffinity purification using the monoclonal antibody against the tag and then purified on a metaloaffinity column.

Any scFv accumulating within the bacteria is purified by isolating and repeatedly washing the inclusion bodies, followed by solublization by e.g. urea or guanidinium and subsequent renaturation.

Alternative possibilities are employing an oligohistidine as the tag, using a stronger promoter instead of lac, i.e. T7, constructing the vector without the leader sequence or introducing a sequence encoding a "tail" of irrelevant sequences into the vector at the 5' end of the scFv. This "tail" should not be biologically active, since its only purpose is the creation of a longer molecule than the native scFv, thus causing a longer retention time in the body.

### EXAMPLE 8:

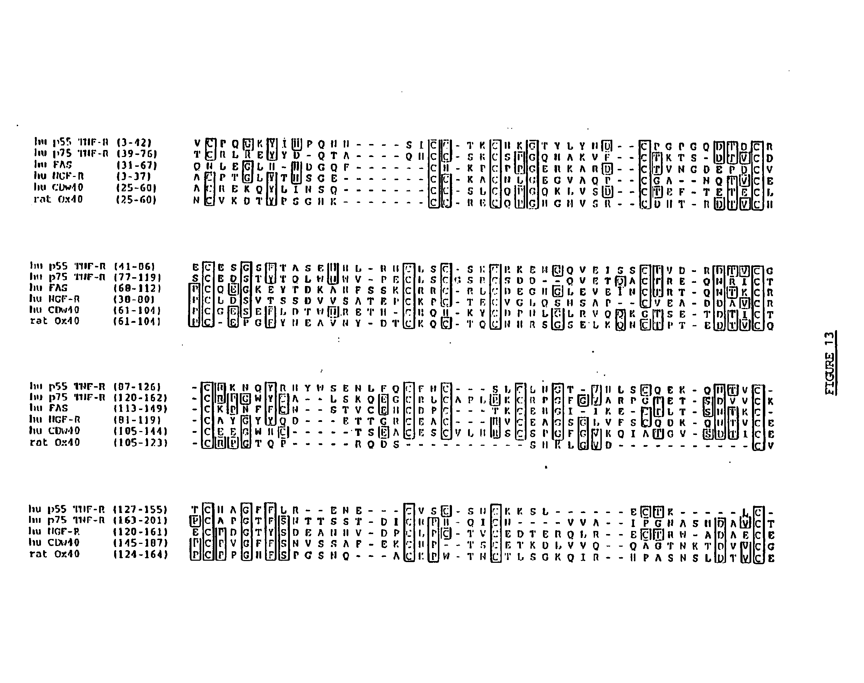

Figure 13 shows the internal cysteine rich repeats in the extracellular domains of the two TNF-Rs and their alignment with the homologous repeats in the extracellular domain of the human FAS, nerve growth factor receptor (NGF) and CDw40, as well as rat Ox40. The amino acid sequences (one letter symbols) are aligned for maximal homology. The positions of the amino acids within the receptors are denoted in the left hand margin.

### EXAMPLE 9: Creation of recombinant DNA molecules comprising nucleotide sequences coding for the active peptides and other molecules and their expression

The peptides and other molecules can also be prepared by genetic engineering techniques and their preparation encompasses all the tools used in these techniques. Thus DNA molecules are provided which comprise the nucleotide sequence coding for such peptides and other biological molecules. These DNA molecules can be genomic DNA, cDNA, synthetic DNA and a combination thereof.

Creation of DNA molecules coding for such peptides and molecules is carried out by conventional means, once the amino acid sequence of these peptides and other molecules has been determined.

Expression of the recombinant proteins can be effected in eukaryotic cells, bacteria or yeasts, using the appropriate expression vectors. Any method known in the art may be employed.

For example, the DNA molecules coding for the peptides or other molecules obtained by the above methods are inserted into appropriately constructed expression vectors by techniques well known in the art (see Maniatis, T. et al., Molecular Cloning: A Laboratory Manual, Cold Spring Harbor Laboratory, Cold Spring Harbor (1982)). Double-stranded cDNA is linked to plasmid vectors by homopolymeric tailing or by restriction linking involving the use of synthetic DNA linkers or blunt-ended ligation techniques.

DNA ligases are used to ligate the DNA molecules and undesirable joining is avoided by treatment with alkaline phosphatase.

In order to be capable of expressing a desired biological substance, i.e. a peptide or protein (hereinafter "protein", for simplicity's sake), an expression vector should comprise also specific nucleotide sequences containing transcriptional and translational regulatory information linked to the DNA coding for the desired protein in such a way as to permit gene expression and production of the protein. First, in order for the gene to be transcribed, it must be preceded by a promoter recognizable by RNA polymerase, to which the polymerase binds and thus initiates the transcription process. There are a variety of such promoters in use, which work with different efficiencies (strong and weak promoters). They are different for prokaryotic and eukaryotic cells.

The promoters that can be used in the present invention may be either constitutive, for example, the int promoter of bacteriophage lambda, the bla promoter of the β-lactamase gene of pBR322, and the CAT promoter of the chloramphenicol acetyl transferase gene of pPR325, etc., or inducible, such as the prokaryotic promoters including the major right and left promoters of bacteriophage lambda (P_{L} and P_{R}), the trp, recA, lacZ, lacI, ompF and gal promoters of E. coli, or the trp-lac hybrid promoter, etc. (Glick, B.R. (1987) J.Ind.Microbiol., 1:277-282).

Besides the use of strong promoters to generate large quantities of mRNA, in order to achieve high levels of gene expression in prokaryotic cells, it is necessary to use also ribosome-binding sites to ensure that the mRNA is efficiently translated. One example is the Shine-Dalgarno (SD) sequence appropriately positioned from the initiation codon and complementary to the 3'-terminal sequence of 16S RNA.

For eukaryotic hosts, different transcriptional and translational regulatory sequences may be employed, depending on the nature of the host. They may be derived from viral sources, such as adenovirus, bovine papilloma virus, Simian virus, or the like, where the regulatory signals are associated with a particular gene which has a high level of expression. Examples are the TK promoter of Herpes virus, the SV40 early promoter, the yeast gal4 gene promoter, etc. Transcriptional initiation regulatory signals may be selected which allow for repression and activation, so that expression of the genes can be modulated.

The DNA molecule comprising the nucleotide sequence coding for the peptides or other molecules of the invention and the operably linked transcriptional and translational regulatory signals is inserted into a vector which is capable of integrating the desired gene sequences into the host cell chromosome. The cells which have stably integrated the introduced DNA into their chromosomes can be selected by also introducing one or more markers which allow for selection of host cells which contain the expression vector. The marker may provide for prototrophy to an auxotropic host, biocide resistance, e.g., antibiotics, or heavy metals, such as copper, or the like. The selectable marker gene can either be directly linked to the DNA gene sequences to be expressed, or introduced into the same cell by co-transfection. Additional elements may also be needed for optimal synthesis of single chain binding protein mRNA. These elements may include splice signals, as well as transcription promoters, enhancers, and termination signals. cDNA expression vectors incorporating such elements include those described by Okayama, H., (1983) Mol. Cell Biol., 3:280.

In a preferred embodiment, the introduced DNA molecule will be incorporated into a plasmid or viral vector capable of autonomous replication in the recipient host. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Preferred prokaryotic vectors include plasmids such as those capable of replication in E. coli, for example, pBR322, ColEl, pSC101, pACYC 184, etc. (see Maniatis et al., (1982) op. cit.); Bacillus plasmids such as pC194, pC221, pT127, etc. (Gryczan, T., The Molecular Biology of the Bacilli, Academic Press, NY (1982)); Streptomyces plasmids including pIJ101 (Kendall, K.J. et al., (1987) J.Bacteriol. 169:4177-83); Streptomyces bacteriophages such as φC31 (Chater, K.F. et al., in: Sixth International Symposium on Actinomycetales Biology, (1986)), and Pseudomonas plasmids (John, J.F., et al. (1986) Rev.Infect.Dis. 8:693-704; and Izaki, K. (1978) Jpn. J. Bacteriol., 33:729-742).

Preferred eukaryotic plasmids include BPV, vaccinia, SV40, 2-micron circle, etc., or their derivatives. Such plasmids are well known in the art (Botstein, D., et al. (1982) Miami Wint. Symp. 19, pp. 265-274; Broach, J.R., in: The Molecular Biology of the Yeast Saccharomyces: Life Cycle and Inheritance, Cold Spring Harbor Laboratory, Cold Spring Harbor, NY, pp. 445-470 (1981); Broach, J.R., (1982) Cell, 28:203-204; Bollon, D.P., et al. (1980) J. Clin. Hematol. Oncol., 10:39-48; Maniatis, T., in: Cell Biology: A Comprehensive Treatise, Vol. 3: Gene Expression, Academic Press, NY, pp. 563-608 (1980)).

Once the vector or DNA sequence containing the construct(s) has been prepared for expression, the DNA construct(s) may be introduced into an appropriate host cell by any of a variety of suitable means: transformation, transfection, conjugation, protoplast fusion, electroporation, calcium phosphate-precipitation, direct microinjection, etc.

Host cells to be used in this invention may be either prokaryotic or eukaryotic. Preferred prokaryotic hosts include bacteria such as E. coli, Bacillus, Streptomyces, Pseudomonas, Salmonella, Serratia, etc. The most preferred prokaryotic host is E. coli. Bacterial hosts of particular interest include E. coli K12 strain 294 (ATCC 31446), E. coli X1776 (ATCC 31537), E. coli W3110 (F⁻, lambda⁻, prototropic (ATCC 27325)), and other enterobacteria such as Salmonella typhimurium or Serratia marcescens and various Pseudomonas species. Under such conditions, the protein will not be glycosylated. The prokaryotic host must be compatible with the replicon and control sequences in the expression plasmid.

Preferred eukaryotic hosts are mammalian cells, e.g. human, monkey, mouse and chinese hamster ovary (CHO) cells, because they provide post-translational modifications to protein molecules including correct folding or glycosylation at correct sites. Also yeast cells can carry out post-translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products and secretes peptides bearing leader sequences (i.e. pre-peptides).

After the introduction of the vector, the host cells are grown in a selective medium, which selects for the growth of vector-containing cells. Expression of the cloned gene sequence(s) results in the production of the desired proteins.

Purification of the recombinant proteins is carried out by any one of the methods known for this purpose.

### Deposit Information

Hybridoma TBP-II 70-2 was deposited with the Collection National de Cultures de Microorganismes, Institut Pasteur (CNCM) on March 12, 1990 and assigned No. I-928.

Hybridoma TBP-II 32-5 was deposited with the CNCM on September 1, 1993 and assigned No. I-1358.

The deposited hybridomas TBP-II 70-2 and TBP-II 32-5 are subclones of hybridomas TBP-II 70 and TBP-II 32, respectively and have identical properties.The production and cloning of these hybridomas has been described in EP-A- 398 327.

## Claims

1. Use of a ligand to a member of the tumor necrosis factor/nerve growth factor (TNF/NGF) receptor family which binds to the region of the C- terminal cysteine loop of such a receptor and which is inhibitory to the signaling for the cytocidal effect of said receptor without blocking the binding of TNF or the respective natural ligand to the receptor, wherein the cysteine loop includes the amino acid sequence cys-163 to thr-179 in the p75 TNF-R (Fig. 5), or a corresponding C-terminal cysteine loop in another member of the TNF/NGF receptor family, for the preparation of a pharmaceutical composition for the treatment of septic shock, cachexia, graft-versus-host disease and autoimmune diseases, in particular rheumatoid arthritis, wherein said ligand is an antibody, a peptide derived therefrom, or a salt or mutein of said antibody.

2. The use according to claim 1, wherein the ligand comprises a ligand to a TNF-R.

3. The use according to claim 1 or 2, wherein the receptor is the p75 TNF-R.

4. The use according to any one of claims 1 to 3, wherein the ligand includes the amino acid sequence for the CDR region of the light chain of monoclonal antibody No. 32 shown in Fig. 12.

5. The use according to any one of claims 1 to 3, wherein the ligand includes the amino acid sequence for the CDR region of the heavy chain of monoclonal antibody No. 57 shown in Fig. 11.

6. The use according any one of claims 1 to 3, wherein the ligand includes the amino acid sequence of an antibody raised against the C-terminal loop of a member of the TNF/NGF receptor family.

7. The use according to any one of claims 1 to 3, wherein the ligand comprises the scFv of monoclonal antibody No. 32 (CNCM 1- 1358) or 70 (CNCM 1-928).

8. The use according to any one of claims 4 to 7, wherein the ligand includes polyethylene glycol side chains.

9. The use according to any one of claims 4 to 7, wherein the ligand is a fused protein comprising said ligand which still binds to the region of the C-terminal cysteine loop of the TNF/NGF receptor and which is still inhibitory to the signaling for the cytocidal effect of said receptor.

10. A ligand as defined in claim I comprising the scFv of monoclonal antibody No. 32 (CNCM 1-1358)

11. A ligand as defined in claim 1, including the amino acid sequence for the CDR region of the light chain of monoclonal antibody No. 32 shown in Fig. 12.

12. A ligand as defined in claim 1, including the amino acid sequence for the CDR region of the heavy chain of monoclonal antibody No. 57 shown in Fig. 11.

13. A DNA molecule encoding a ligand according to any of the claims 10 to 12, capable of expressing such a ligand.

14. A DNA molecule hybridizing to a DNA molecule according to claim 13 and capable of expressing a ligand according to any of the claims 10 to 12.

15. A replicable expression vehicle comprising a DNA molecule according to claim 13 or 14, and capable of expressing a ligand according to any of the claims 10 to 12 in a transformant host cell.

16. A host cell transformed with the replicable expression vehicle of claim 15.

17. A host cell according to claim 16 which is a prokaryotic cell.

18. A host cell according to claim 16 which is a eukaryotic cell.

19. A process for the production of a recombinant ligand according to any of the claims 10 to 12, comprising culturing a transformed host cell according to any one of claims 16 to 18 and recovering the recombinant ligand.

20. A pharmaceutical composition comprising a ligand according to any of the claims 10 to 12.

21. A ligand according to any of the claims 10 to 12 which includes polyethylene glycol side chains which still binds to the region of the C-terminal cysteine loop of the TNF/NGF receptor and which is still inhibitory to the signaling for the cytocidal effect of said receptor.

## Patentansprüche

1. Verwendung eines Liganden für ein Mitglied der Tumornekrosefaktor/Nervenwachstumsfaktor (TNF/NGF)-Rezeptorfamilie, welcher an den Bereich der C-terminalen Cysteinschleife dieses Rezeptors bindet und welcher auf die Signalgebung für den cytotoxischen Effekt dieses Rezeptors inhibitorisch wirkt, ohne das Binden von TNF oder des entsprechenden natürlichen Liganden an den Rezeptor zu blockieren, wobei die Cysteinschleife die Aminosäuresequenz cys-163 bis thr-179 des p75-TNF-R (Fig. 5) oder eine entsprechende C-terminale Cysteinschleife eines anderen Mitglieds der TNF/NGF-Rezeptorfamilie umfasst, zur Herstellung eines Arzneimittels für die Behandlung von septischem Schock, Kachexie, Transplantat-gegen-Empfänger-Erkrankung und Autoimmunkrankheiten, insbesondere Arthritis rheumatica, wobei der Ligand ein Antikörper, ein davon abgeleitetes Peptid oder ein Salz oder Mutein des Antikörpers ist.

2. Verwendung nach Anspruch 1, wobei der Ligand einen Liganden eines TNF-R umfasst.

3. Verwendung nach Anspruch 1 oder 2, wobei der Rezeptor der p75 TNF-R ist.

4. Verwendung nach einem der Ansprüche 1 bis 3, wobei der Ligand die in Figur 12 dargestellte Aminosäuresequenz für die CDR-Region der leichten Kette des monoclonalen Antikörpers Nr. 32 einschließt.

5. Verwendung nach einem der Ansprüche 1 bis 3, wobei der Ligand die in Figur 11 dargestellte Aminosäuresequenz für die CDR-Region der schweren Kette des monoclonalen Antikörpers Nr. 57 einschließt.

6. Verwendung nach einem der Ansprüche 1 bis 3, wobei der Ligand die Aminosäuresequenz eines Antikörpers einschließt, der gegen die C-terminale Schleife eines Mitglieds der TNF/NGF-Rezeptorfamilie gerichtet ist.

7. Verwendung nach einem der Ansprüche 1 bis 3, wobei der Ligand das scFv des monoclonalen Antikörpers Nr. 32 (CNCM I - 1358) oder 70 (CNCM I - 928) umfasst.

8. Verwendung nach einem der Ansprüche 4 bis 7, wobei der Ligand Polyethylenglycol-Seitenketten einschließt.

9. Verwendung nach einem der Ansprüche 4 bis 7, wobei der Ligand ein Fusionsprotein ist, das den Liganden umfasst, der immer noch an den Bereich der C-terminalen Cysteinschleife des TNF/NGF-Rezeptors bindet und immer noch auf die Signalgebung für den cytotoxischen Effekt des Rezeptors inhibitorisch wirkt.

10. Ligand wie in Anspruch 1 definiert, umfassend das scFv des monoclonalen Antikörpers Nr. 32 (CNCMI- 1358).

11. Ligand wie in Anspruch 1 definiert, der die in Figur 12 dargestellte Aminosäuresequenz für die CDR-Region der leichten Kette des monoclonalen Antikörpers Nr. 32 einschließt.

12. Ligand wie in Anspruch 1 definiert, der die in Figur 11 dargestellte Aminosäuresequenz für die CDR-Region der schweren Kette des monoclonalen Antikörpers Nr. 57 einschließt.

13. DNA-Molekül, das einen Liganden nach einem der Ansprüche 10 bis 12 codiert und zur Expression eines solchen Liganden geeignet ist.

14. DNA-Molekül, das an ein DNA-Molekül nach Anspruch 13 hybridisiert und zur Expression eines Liganden nach einem der Ansprüche 10 bis 12 geeignet ist.

15. Replizierbares Expressionsvehikel, das ein DNA-Molekül nach Anspruch 13 oder 14 umfasst und das zur Expression eines Liganden nach einem der Ansprüche 10 bis 12 in einer transformierten Wirtszelle geeignet ist.

16. Wirtszelle, transformiert mit dem replizierbaren Expressionsvehikel nach Anspruch 15.

17. Wirtszelle nach Anspruch 16, die eine prokaryontische Zelle ist.

18. Wirtszelle nach Anspruch 16, die eine eukaryontische Zelle ist.

19. Verfahren zur Herstellung eines rekombinanten Liganden nach einem der Ansprüche 10 bis 12, umfassend die Züchtung einer transformierten Wirtszelle nach einem der Ansprüche 16 bis 18 und die Gewinnung des rekombinanten Liganden.

20. Arzneimittel, umfassend einen Liganden nach einem der Ansprüche 10 bis 12.

21. Ligand nach einem der Ansprüche 10 bis 12, der Polyethylenglycol-Seitenketten einschließt und immer noch an den Bereich der C-terminalen Cysteinschleife des TNF/NGF-Rezeptors bindet und immer noch auf die Signalgebung für den cytotoxischen Effekt des Rezeptors inhibitorisch wirkt.

## Revendications

1. Utilisation d'un ligand d'un élément de la famille du récepteur du facteur de nécrose tumorale/facteur de croissance nerveuse (TNF/NGF), qui se lie à la région de la boucle cystéine C-terminale d'un tel récepteur, et qui est inhibiteur de signal pour l'effet cytocide dudit récepteur, sans blocage de la liaison, au récepteur, du TNF ou du ligand naturel correspondant, où la boucle cystéine comprend la séquence des acides aminés cys-163 à thr-179 dans le p75 TNF-R (Fig. 5), ou une boucle cystéine C-terminale correspondante dans un autre élément de la famille du récepteur du TNF/NGF, pour la préparation d'une composition pharmaceutique destinée au traitement du choc septique, de la cachexie, de la réaction du greffon contre l'hôte et des maladies auto-immunes, en particulier la polyarthrite rhumatoïde, où ledit ligand est un anticorps, un peptide qui en dérive, ou un sel ou une mutéine dudit anticorps.

2. Utilisation selon la revendication 1, dans laquelle le ligand comprend un ligand d'un TNF-R.

3. Utilisation selon la revendication 1 ou 2, dans laquelle le récepteur est le p75 TNF-R.

4. Utilisation selon l'une quelconque des revendications 1 à 3, dans laquelle le ligand comprend la séquence d'acides aminés pour la région CDR de la chaîne lègère de l'anticorps monoclonal N° 32 présentée sur la Fig. 12.

5. Utilisation selon l'une quelconque des revendications 1 à 3, dans laquelle le ligand comprend la séquence d'acides aminés pour la région CDR de la chaîne lourde de l'anticorps monoclonal N° 57, présentée sur la Fig. 11.

6. Utilisation selon l'une des revendications 1 à 3, dans laquelle le ligand comprend la séquence d'acides aminés d'un anticorps dirigé contre la boucle C-terminale d'un élément de la famille du récepteur TNF/NGF.

7. Utilisation selon l'une quelconque des revendications 1 à 3, dans laquelle le ligand comprend le scFv de l'anticorps monoclonal N° 32 (CNCM 1-1358) ou 70 (CNCM I-928).

8. Utilisation selon l'une quelconque des revendications 4 à 7, dans laquelle le ligand comprend des chaînes latérales polyéthylèneglycol.

9. Utilisation selon l'une quelconque des revendications 4 à 7, dans laquelle le ligand est une protéine fusionnée comprenant ledit ligand, qui se lie encore à la région de la boucle cystéine C-terminale du récepteur du TNF/NGF, et qui est encore inhibiteur du signal de l'effet cytocide dudit récepteur.

10. Ligand selon la revendication 1, comprenant le scFv de l'anticorps monoclonal N° 32 (CNCM I-1358).

11. Ligand selon la revendication 1, comprenant la séquence d'acides aminés pour la région CDR de la chaîne lègère de l'anticorps monoclonal N° 32 présentée sur la Fig. 12.

12. Ligand selon la revendication 1, comprenant la séquence d'acides aminés pour la région CDR de la chaîne lourde de l'anticorps monoclonal N° 57 présentée sur la Fig. 11.

13. Molécule d'ADN codant pour un ligand selon l'une quelconque des revendications 10 à 12, capable d'exprimer un tel ligand.

14. Molécule d'ADN qui s'hybride à une molécule d'ADN selon la revendication 13 et capable d'exprimer un ligand selon l'une quelconque des revendications 10 à 12.

15. Véhicule d'expression réplicable comprenant une molécule d'ADN selon la revendication 13 ou 14, et capable d'exprimer un ligand selon l'une quelconque des revendications 10 à 12 dans une cellule hôte transformante.

16. Cellule hôte transformée avec le véhicule d'expression réplicable selon la revendication 15.

17. Cellule hôte selon la revendication 16, qui est un cellule procaryote.

18. Cellule hôte selon la revendication 16, qui est une cellule eucaryote.

19. Procédé pour la production d'un ligand recombinant selon l'une quelconque des revendications 10 à 12, comprenant la culture d'une cellule hôte transformée selon l'une quelconque des revendications 16 à 18, et la récupération du ligand recombinant.

20. Composition pharmaceutique comprenant un ligand selon l'une quelconque des revendications 10 ou 12.

21. Ligand selon l'une quelconque des revendications 10 à 12, qui comprend des chaînes latérales polyéthylèneglycol, qui se lient encore à la région de la boucle cystéine C-terminale du récepteur du TNF/NGF, et qui est encore inhibiteur du signal de l'effet cytocide dudit récepteur.
